(19) Europäisches Patentamt
European Patent Office
Office européen des brevets

(11) Publication number: **0 174 071**
**A2**

# (12) EUROPEAN PATENT APPLICATION

(21) Application number: 85304805.6

(22) Date of filing: 05.07.85

(51) Int. Cl.[4]: C 07 D 211/90
A 61 K 31/44

(30) Priority: 28.07.84 GB 8419332

(43) Date of publication of application: 12.03.86 Bulletin 86/11

(84) Designated Contracting States: AT BE CH DE FR GB IT LI LU NL SE

(71) Applicant: FISONS plc
Fison House Princes Street
Ipswich Suffolk IP1 1QH(GB)

(72) Inventor: Baxter, Andrew John Gilby
78 Mount Pleasant
Keyworth Nottinghamshire(GB)

(72) Inventor: Dixon, John
Church Farmhouse Main Street Great Dalby
Nr Melton Mowbray Leicestershire(GB)

(72) Inventor: Gould, Kenneth John
3A Turvey Lane
Long Whatton Leicestershire(GB)

(72) Inventor: Fuher, John
62 Wilmington Avenue
Alvaston Derbyshire(GB)

(74) Representative: Craig, Christopher Bradberry et al,
Fisons plc 12 Derby Road
Loughborough Leicestershire LE11 0BB(GB)

## (54) Dihydropyridine derivatives and their production, formulation and use as pharmaceuticals.

(57) There are described compounds of formula I,

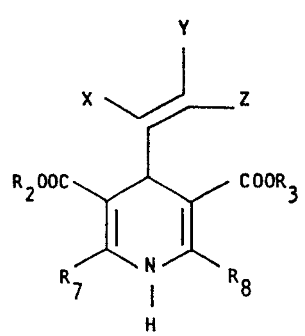

I

in which $R_2$ and $R_3$, which may be the same or different, each represent alkyl C1 to 6 or cycloalkyl C3 to 8,

$R_7$ and $R_8$, which may be the same or different, each represent alkyl C1 to 6 optionally substituted by halogen, and

X, Y and Z, which may be the same or different, are each hydrogen, halogen, nitro or alkyl C1 to 6 optionally substituted by halogen, or Z is as defined above and X and Y, together with the carbon atoms to which they are attached, form a C5 to 8 carbocyclic ring, other than a benzene ring,

provided that when $R_2$, $R_3$, $R_7$ and $R_8$ are all methyl and X and Z are hydrogen; or when $R_2$ and $R_3$ are both ethyl, $R_7$ and $R_8$ are both methyl and X and Z are hydrogen; then Y is other than methyl.

There are also described methods for making the compounds and their formulation and use as pharmaceuticals, e.g. for the treatment of cardiovascular conditions.

EP 0 174 071 A2

0174071

DIHYDROPYRIDINE DERIVATIVES AND THEIR PRODUCTION, FORMULATION AND USE AS PHARMATEUTICALS

This invention relates to new compounds, methods for their preparation and compositions containing them.

A wide variety of dihydropyridines, e.g. those described in US Patent No 3,325,505, have been described as being useful as pharmaceuticals and some, notably nifedipine, have been sold for this use.

We have now found a new group of pyridine derivatives which have pharmacological activity.

According to the invention we provide compounds of formula I,

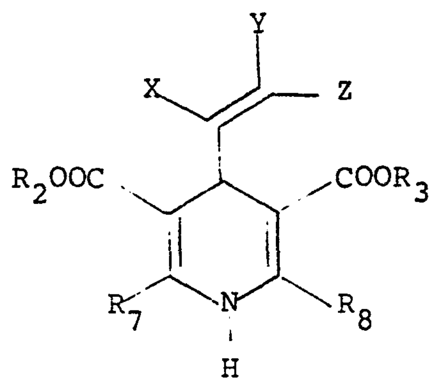

I

in which $R_2$ and $R_3$, which may be the same or different, each represent alkyl C1 to 6 or cycloalkyl C3 to 8,

$R_7$ and $R_8$, which may be the same or different, each represent alkyl C1 to 6 optionally substituted by halogen, and

X, Y and Z, which may be the same or different, are each hydrogen, halogen, nitro or alkyl C1 to 6 optionally substituted by halogen, or Z is as defined above and X and

Y, together with the carbon atoms to which they are attached, form a C5 to 8 carbocyclic ring, other than a benzene ring,

provided that when $R_2$, $R_3$, $R_7$ and $R_8$ are all methyl and X and Z are hydrogen; or when $R_2$ and $R_3$ are both ethyl, $R_7$ and $R_8$ are both methyl and X and Z are hydrogen; then Y is other than methyl.

According to the invention we also provide the compounds of formula I for use as pharmaceuticals.

According to the invention we further provide a process for the production of a compound of formula I which comprises

a) reaction of a compound of formula II,

$YZC=CXCHO$ II

either with a compound of formula III or with compounds of formulae III and IV,

$R_2OOCCH=C(R_7)NH_2$ III

$R_3OOCCH_2COR_8$ IV

in which formulae Y,Z,X, $R_2$, $R_3$, $R_7$ and $R_8$ are as defined above,

b) reaction of a compound of formula V,

$YZC=CX-CH=C(COOR_3)COR_8$ V

in which Y, Z, X, $R_3$ and $R_8$ are as defined above, with a compound of formula III, or

c) production of an optical isomer of a compound of

formula I by resolution of a mixture of optical isomers of the compound.

The reaction of process a) may be carried out by subjecting the compounds of formulae II, III and IV to an elevated temperature, eg of from about 20° and 140°C in the presence or absence of a suitable solvent, eg a lower alkanol or dimethylformamide. Alternatively the compound of formula II may be reacted with two moles of the compound of formula III to produce a symmetrical compound.

Processes b) may be carried out under similar conditions to process a).

Process c) may be carried out using conventional processes known *per se*.

The starting materials for the above processes are either known, or if they are not specifically known they may be made by processes described in the Examples, or they may be made from known compounds using one or more process steps which are known *per se* or are analogous to those described in the Examples.

The compounds of formula I and the intermediates therefor may be isolated from their reaction mixtures using conventional processes, eg crystallisation or chromatography.

The compounds of formula I, are useful because they

exhibit pharmacological properties in animals. More particularly they block the entry of calcium into vascular and cardiac muscle leading to falls in blood pressure, inotropy and heart rate. They are active in the following systems:-

a) Relaxation of contracted vascular smooth muscle. Van Breeman, Aaronson, Loutzenhiser and Meisheri, Chest, 78, Supplement, 157-165, 1980.

b) Reduction of inotropy and chronotropy of isolated atria. Henry, Excerpta Med. Int. Congr. Ser., 474, 14-23, 1979.

c) Reduction of blood pressure and increase cardiac output in anaesthetised dogs. Hirakawa, Ito, Kondo, Watanbe, Hiei, Banno & Hyase, Arzneim-Forsch, 22, 344-349, 1972.

d) Reduction of blood pressure in conscious dogs when given by the intravenous and oral routes. Newman, Bishop, Peterson, Leroux & Horowitz, J Pharm. Exp. Ther. 201, 723-730, 1977.

The compounds are thus indicated for use in the treatment of renovascular, malignant or essential hypertension (including hypertensive emergencies), pulmonary hypertension, vasospastic angina, chronic stable angina and congestive heart failure. Other indications are the treatment of renal failure, cardiac arrhythmias,

hypertrophic cardiomyopathy, cerebrovascular diseases (including cerebral haemorrhage, ischaemia and vasospasm, migraine, altitude sickness and hearing loss), peripheral vascular diseases (including Raynaud's syndrome, intermittent claudication and digital ulceration); use as a cardioplegic agent during surgery, e.g. in cardiopulmonary bypass, and for the treatment of, and protection against, myocardial infarction and ischaemia.

By virtue of their ability to inhibit calcium entry into other cells and tissues the compounds are also indicated in the treatment of thrombosis, atherosclerosis, respiratory diseases (including asthma and bronchitis) glaucoma, aldosteronism, uterine hypermotility and for the relief of oesophageal and skeletal muscle spasm.

For the above uses the dosage will depend upon the compound used, the route of administration and the effect desired, but in general will be in the range of 0.1-10mg per kilogram body weight per day. For man the indicated total daily dose will be from about 5-500mg, preferably from 5 to 200mg and more preferably from 5 to 100mg, which may be administered preferably once daily, or in divided doses of about 1-200mg, preferably 2 to 25mg, e.g. 2 to 4 times per day.

The compounds of formula I are advantageous in that they possess greater potency (e.g. with respect to

hypotensive and direct negative chronotropic effects), produce a lower level of reflex tachycardia, are more selective (e.g. for vascular smooth muscle vs cardiac muscle), produce less depression of cardiac contractility, are longer acting, are more readily absorbed or less readily metabolised, are more easily formulated, possess less, or less undesirable, side effects, are more stable or have other more beneficial properties than known compounds of similar structure.

The compounds of the invention may be administered by a wide variety of routes and may act systemically or locally. Thus the compounds may be administered by oral or nasal inhalation to the lung, to the buccal cavity, oesophageally, rectally, topically to the skin, the eye or to other available surfaces of the body; by injection, e.g. intravenously, intramuscularly, intraperitoneally, or by surgical implant.

We prefer $R_2$ and $R_3$ to be selected from methyl, ethyl, 1-methylethyl, cyclobutyl and cyclopentyl. We particularly prefer $R_2$ to be 1-methylethyl, cyclobutyl or cyclopentyl and $R_3$ to be methyl. We prefer $R_7$ and $R_8$ to be selected from methyl and monofluoromethyl, and especially for $R_7$ to be methyl and $R_8$ to be monofluoromethyl. Z may be fluoro, chloro, bromo, $-CF_3$, $-NO_2$ or methyl; X may be fluoro, chloro, methyl

or hydrogen; Y may be $-CF_3$, fluoro, chloro, methyl, $-NO_2$ or hydrogen; or X and Y may together form a $-(CH_2)_n-$ chain in which n is 3 or 4.

When X and Y, together with the carbon atoms to which they are attached, form a carbocyclic ring that ring may be a bridged ring, e.g. a ring of formula,

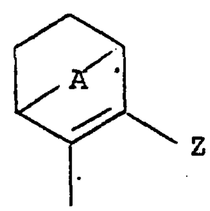

in which Z is as defined above, and A is $-CH_2-$ or $-CH_2CH_2-$.

According to our invention we also provide a pharmaceutical composition comprising preferably less than 80%, more preferably less than 50%, e.g. 1 to 20%, by weight of a compound of formula I, in admixture with a pharmaceutically acceptable adjuvant, diluent or carrier.

Thus the compound may be put up as a tablet, capsule, dragee, suppository, suspension, solution, injection, implant, a topical, e.g. transdermal, preparation such as a gel, cream, ointment, aerosol or a polymer system, or an inhalation form, e.g. an aerosol or a powder formulation.

We prefer compositions which are designed to be taken oesophageally and to release their contents in the

gastrointestinal tract. Thus we prefer tablets which may, for example, be made by direct compression. In such a process the active ingredient is mixed with one or more of modified forms of starch, calcium phosphate, a sugar, e.g. lactose, microcrystalline cellulose and/or other directly compressible excipients, together with lubricant(s), e.g. stearic acid or magnesium stearate, flow aid(s), e.g. talc or colloidal silicon dioxide, and disintegrant(s), e.g. starch, substituted sodium carboxymethyl cellulose, cross linked sodium carboxy methyl cellulose, carboxy methyl starch and cross linked polyvinylpyrrolidone. Tablets are then formed by direct compression, and may be sugar or film coated, e.g. with hydroxpropylmethylcellulose.

Alternatively the active ingredient may be granulated before tabletting. In such cases the active ingredient is mixed with one or more of starch, calcium phosphate, a sugar, e.g. lactose, microcrystalline cellulose or other suitable excipients and granulated with a binder such as starch, pregelled starch, polyvinylpyrrolidone, gelatine, a modified gelatine, or a cellulose derivative, e.g. hydroxypropylmethylcellulose. The mass is then dried, sieved and mixed with lubricant(s), flow aid(s) and disintegrant(s), such as described in the previous paragraph. Tablets are then formed by compression of the

granules, and may be sugar or film coated, e.g. with hydroxypropylmethylcellulose.

As a further alternative a powder, blend or granules, such as are described above as intermediates in tabletting, may be filled into a suitable, e.g. gelatine, capsule.

In order to improve the bioavailability, or decrease variability of availability, of the active ingredient the compound may be:-

a) dissolved in a suitable solvent, e.g. polyethylene glycol, Gelucaire, arachis oil, a (hydrogenated) vegetable oil or beeswax and the solution is then filled into a gelatine capsule,

b) produced as a spray-dried or freeze-dried form prior to mixing with other excipients,

c) milled and/or micronised to produce a powder with a large surface area prior to mixing with other excipients,

d) made into a solution and distributed over an inert excipient having a large surface area, e.g. colloidal silicon dioxide. The solvent is evaporated and further excipients added,

e) formed into a complex with cyclodextrin prior to mixing with other excipients. This complex also assists in increasing light stability, or

f) made into a solid solution or co-precipitated, e.g.

with polyvinylpyrrolidone, polyethyleneglycol, modified cellulose, hydroxypropylmethylcellulose, urea or a sugar prior to mixing with further excipients.

The compounds, either in their normal form or in a modified form, e.g. as described immediately above, may be formulated in a controlled release form. Thus the compound may be dispersed, or contained in, a polymer matrix formed from, for example, ethylcellulose, hydroxypropylmethylcellulose or an acrylate/methacrylate polymer. Alternatively the compound may be formulated as a tablet or beads which are surrounded by a semi-permeable membrane, e.g. shellac, ethylcellulose or an acrylate/methacrylate polymer.

The compounds of this invention may be given in combination with other pharmaceutically active compounds, e.g. diuretics, beta-blockers, antihypertensives or inotropic agents. The dosage of the other pharmaceutically active compound can be that conventionally used when the compound is administered on its own, but is preferably somewhat lower. To illustrate these combinations, a compound of this invention effective in the range, e.g. 5-100 milligrams per day, can be combined at levels ranging. e.g. from 1-200 milligrams per day with the following beta-blockers, antihypertensives and diuretics in dose ranges per day as indicated:

hydrochlorothiazide (15-200mg), chlorothiazide (125-2000mg), ethacrynic acid (15-100mg), amiloride (5-20mg), furosemide (5-80mg), propanolol (20-480mg), timolol (5-50mg), captopril (10-500mg), methyldopa (65-2000mg) or digoxin (0.1-0.5mg). In addition, the triple drug combinations of hydrochlorothiazide (15-200mg) plus amiloride (5-20mg) plus a compound of this invention (3-200mg) and hydrochlorothiazide (15-200mg) plus timolol (5-200mg) plus a compound of this invention (3-200mg), are provided. The above dose ranges may be adjusted on a unit basis as necessary to permit divided daily dosage. Also, the dose may vary depending on the severity of the disease, weight of patient and other factors which a person skilled in the art will recognise.

Certain of the compounds of formula I are assymetric and exhibit optical isomerism. Such compounds may be separated into their optical isomers using process c) or may be made by stereospecific syntheses using conventional techniques known *per se*.

The invention therefore provides the compounds as their individual optical isomers, and racemic or other mixtures of the individual isomers.

Certain of the compounds of the invention can form solvates, e.g. hydrates or alcoholates, and certain of the compounds are light sensitive and should therefore be

produced, handled, stored and formulated in such a manner that they are not subjected to degrading amounts of light of the appropriate wavelengths.

The invention is illustrated, but in no way limited by the following Examples in which temperatures are in degrees centigrade.

Example 1

3-Methyl 5-(1-methylethyl) 4-(2-chloro-1-cyclohexenyl)-2-(fluoromethyl)-1,4-dihydro-6-methyl-3,5-pyridinedicarboxylate

2-Chloro-1-cyclohexene-1-carboxaldehyde (2.9g, 20 mmoles), methyl 4-fluoro-3-oxobutanoate (2.7g, 20 mmoles) and 1-methylethyl 3-amino-2-butenoate (2.88g, 20 mmoles) in dried tert-butanol (40ml) was heated with stirring under nitrogen at 35° for 96 hours. The solvent was removed *in vacuo* and the resulting red oil was purified by HPLC using 20% ethyl acetate in petroleum ether (60-80°) as eluant. Pure fractions were combined and evaporated to dryness and the title compound (0.48g) obtained by crystallization from hexane. mp 118-9°.

Example 2

3-Methyl 5-(1-methylethyl) 4-(2-chloro-1-cyclopentenyl)-2-(fluoromethyl)-1,4-dihydro-6-methyl-3,5-pyridinedicarboxylate

2-Chloro-1-cyclopentene-1-carboxaldehyde (3.1g, 23.7

mmoles), methyl 4-fluoro-3-oxobutanoate (3.18g, 23.7 mmoles) and 1-methylethyl 3-amino-2-butenoate (3.4g, 23.7 mmoles) in dried tert-butanol (40ml) was heated with stirring under nitrogen at 45° for 15 hours. The solvent was removed in vacuo and the resulting red oil was purified by HPLC using 20% ethyl acetate in petroleum ether (60-80°) as eluant. Pure fractions were combined and evaporated to dryness and the title compound (2.75g) obtained by crystallization from cyclohexane. mp 133-35°.

Example 3

3-Methyl 5-(1-methylethyl) 4-(2-chloro-1-cyclohexenyl)-1,4-dihydro-2,6-dimethyl-3, 5-pyridinedicarboxylate

2-Chloro-1-cyclohexene-1-carboxaldehyde (5.76g, 40 mmoles), methyl 3-oxobutanoate (4.64g, 40 mmoles) and 1-methylethyl 3-amino-2-butenoate (5.72g, 40 mmoles) were heated with stirring at 95° for 2.5 hours. The reaction mixture was dissolved in ethyl acetate and chromatographed by preparative HPLC eluting with petroleum ether (60-80°)/ethyl acetate mixtures. Pure fractions were combined and evaporated to dryness. The title compound (0.19g) was obtained by crystallization from cyclohexane. mp 133-4°.

Example 4

3-Methyl 5-(1-methylethyl) 2-(fluoromethyl)-1,4-dihydro-6-methyl-4-(2-methyl-1-cyclohexenyl)-3,5-pyridinedicarboxylate

2-Methyl-1-cyclohexene-1-carboxaldehyde (0.7g, 5.6mmoles), methyl 4-fluoro-3-oxobutanoate (0.76g, 5.6mmoles) and 1-methylethyl 3-amino-2-butenoate (0.81g, 5.6mmoles) were heated with stirring under $N_2$ at 60$^{o}$ for 16 hours. The reaction mixture was cooled and then chromatographed on silica eluting with ethyl acetate/petroleum ether (60-80$^{o}$) mixtures. Pure fractions were combined and evaporated and the title compound (0.13g) was obtained by crystallisation from petroleum ether (60-80$^{o}$) mp 136-7$^{o}$.

Example 5

3-Methyl 5-(1-methylethyl) 4-(2,2-dichloroethenyl)-2-(fluoromethyl)-1,4-dihydro-6-methyl-3,5-pyridinedicarboxylate and bis(1-methylethyl) 4-(2,2-dichloroethenyl)-1,4-dihydro-2,6-dimethyl-3,5-pyridinedicarboxylate

1-Methylethyl 3-amino-2-butenoate (1.1g, 8mmoles) was added to a stirred mixture of 3,3-dichloropropenal (1.0g, 8mmoles) and methyl 4-fluoro-3-oxobutanoate (1.1g, 8mmoles). After 1 hour at room temperature and 2 hours at 95$^{o}$, the reaction mixture was cooled and then purified by chromatography on silica eluting with ethyl acetate/petroleum ether (60-80$^{o}$) mixtures. The first

eluted product was the 2-(fluoromethyl)-6-methyl-dihydropyridine which was crystallised from petroleum ether (60-80°) to give a white solid (0.35g) mp 119-20°. The second eluted product was the 2,6-dimethyldihydropyridine which was crystallised from petroleum ether (60-80°) to give a white solid (0.32g) mp 123-4°.

Example 6

3-Methyl 5-(1-methylethyl) 2-(fluoromethyl)-1,4-dihydro-6-methyl-4-(1,2,2-trichloroethenyl)-3,5-pyridinedicarboxylate

2,3,3-Trichloropropenal (0.62g, 3.8mmoles), methyl 4-fluoro-3-oxobutanoate (0.52g, 3.8mmoles) and 1-methylethyl 3-amino-2-butenoate (0.55g, 3.8mmoles) were heated under $N_2$ for 2 hours at 95°. The cooled residue was purified by chromatography on silica eluting with ethyl acetate/petroleum ether (60-80°) mixtures. Pure fractions were combined, the solvent evaporated and the title compound (0.196g) obtained by crystallisation from petroleum ether (60-80°) mp 144-6°.

Example 7

3-Methyl 5-(1-methylethyl) 4-(bicyclo[2.2.2]oct-2-en-2-yl)-2-(fluoromethyl)-1,4-dihydro-6-methyl-3,5-pyridine dicarboxylate

Bicyclo[2.2.2]oct-2-en-2-carboxaldehyde (0.65g,

4.8mmoles), methyl 4-fluoro-3-oxobutanoate (0.75g,5.6mmoles) and 1-methylethyl 3-amino-2-butenoate (0.78g, 5.4mmoles) were heated at 95$^{o}$ for 3.5 hours. The cooled reaction mixture was purified by chromatography on silica eluting with ethyl acetate/ petroleum ether (60-80$^{o}$) mixtures to give the title compound.

$M^+$ 337; nmr ($CDCl_3$) $\delta$ 5.8(s,1H) and 4.5(s,1H).

Example 8

3-Methyl 5-(1-methylethyl) 4-(3-chlorobicyclo [2.2.2]-oct-2-en-2-yl)-2-(fluoromethyl)-1,4-dihydro-6-methyl-3,5-pyridinedicarboxylate

3-Chlorobicyclo[2.2.2]oct-2-en-2-carboxaldehyde (1.4g,8.2mmoles), methyl 4-fluoro-3-oxobutanoate (1.1g 8.2mmoles) and 1-methylethyl 3-amino-2-butenoate (1.2g, 8.2mmoles) were heated at 95$^{o}$ under nitrogen for 5.5 hours. The cooled reaction mixture was purified by chromatography twice on silica eluting with methylene chloride/ethyl acetate and tetrahydrofuran/petroleum ether (60-80$^{o}$) mixtures. Crystallisations from petroleum ether (60-80$^{o}$) gave the title compound (0.04g) mp 137-8$^{o}$.

Example 9

3-Methyl 5-(1-methylethyl) 4-(2-bromo-1-cyclohexenyl)--2-(fluoromethyl)-1,4-dihydro-6-methyl-3,5-pyridinedicarboxylate

Prepared by the method of Example 1 and obtained as pale yellow crystals, mp 103-5°.

Example 10

3-Methyl 5-(1-methylethyl) 2-(fluoromethyl)-1,4 -dihydro-6-methyl-4-(2-(trifluoromethyl)-1-cyclohexenyl)-3,5 -pyridinedicarboxylate

Prepared by the method of Example 1 and obtained as an oil.

$M^+$419; nmr ($CDCl_3$)$\delta$6.4(d,1H), 5.6 (q,2H) and 5.38(m,1H).

Example A

(2,4,6-Tris(1-methylethyl)benzenesulphonyl)(bicyclo [2.2.2]octan-2-ylidene)hydrazine

Bicyclo[2.2.2]octan-2-one (2g, 16mmoles), (2,4,6-tris(1-methylethyl)benzenesulphonyl)hydrazine (4.8g, 16mmoles) and conc.hydrochloric acid (0.2ml) in ethanol (30ml) were heated briefly at reflux and then cooled. Filtration gave the desired hydrazine (5.1g).

$M^+$ 404.

Example B

Bicyclo[2.2.2]oct-2-ene-2-carboxaldehyde

n-Butyl lithium (13.6ml of 1.6M in hexane, 22mmoles) was added over 3 minutes to a stirred solution at -73° under $N_2$ of (2,4,6-tris(1-methylethyl)benenesulphonyl) (bicyclo[2.2.2]octan-2-ylidene)hydrazine (2.2g, 5.4mmoles)

in N,N,N',N',-tetramethyl-1,2-ethanediamine (10ml) and 1,2-dimethoxyethane (50ml). After 15 minutes at -73°, the solution was warmed to -25° and then 0°. Dimethylformamide (7ml) was added and the mixture allowed to reach room temperature. After 30 minutes the reaction mixture was poured onto 10% sulphuric acid and, after 15 minutes, the product was extracted with ethyl acetate (3X). The organic extracts were washed with brine (X 3), saturated sodium hydrogencarbonate (X 3) and brine (X 3), dried ($MgSO_4$) at the solvent evaporated. Chromatography on silica eluting with ethyl acetate/petroleum ether (60-80°) mixtures gave the desired aldehyde (0.56g).

$M^+$ 136; nmr ($CDCl_3$) $\delta$ 9.42(s,1H) and 7.32 (d,1H).

Example C

3-(Hydroxymethylene)bicyclo[2.2.2]octan-2-one

A mixture of bicyclo[2.2.2]octan-2-one (2g, 16mmoles), ethyl formate (1.79g, 24mmoles), sodium metal (0.38g, 16 mmoles), ethanol (1ml) and ether (50ml) was stirred at room temperature for 16 hours. Ethanol (2ml) was added and the stirring continued for 1 hour. The reaction mixture was poured into brine and extracted with ether (X 2). The aqueous layer was acidified with 10% hydrochloric acid and the product was extracted with ether (X 3). This ethereal extract was washed with brine, dried ($MgSO_4$) and the solvent removed to give the title

compound as an oil (1.45g).

$M^+$152; nmr ($CDCl_3$)δ 7.1(s,1H)

Example D

3-[(1-Methylethoxy)methylene]bicyclo[2.2.2] octan-2-one

3-(Hydroxymethylene)bicyclo[2.2.2]octan-2-one (1.45g) was heated at reflux with azeotropic distillation in benzene (40ml) and 2-propanol (40ml) containing a catalytic quantity of 4-methylbenzenesulphonic acid. After 50ml had distilled over, the reaction mixture was cooled and then treated with solid potassium carbonate. After filtration and removal of solvent, the residue was purified by chromatography on silica eluting with ethyl acetate/petroleum ether (60-80°) mixtures to give the title compound as an oil (1.7g).

$M^+$ 194; nmr ($CDCl_3$)δ 7.28 (s,1H).

Example E

3-Chlorobicyclo[2.2.2]oct-2-ene-2-carboxaldehyde

3-[(1-Methylethoxy)methylene]bicyclo[2.2.2] octan-2-one (1.7g, 8.8mmoles) in ether (20ml) was added over 2 minutes to a stirred suspension of phosphorous pentachloride (3.64g, 17.5mmoles) at -50° under nitrogen in ether (50ml). After stirring at -50° for 20 minutes and -15° for 15 minutes, brine was cautiously added. After 15 minutes, the ether layer was separated and the

aqueous layer extracted with ether (X 3). The combined ethereal extracts were dried ($MgSO_4$) and the solvent evaporated to give the title aldehyde (1.2g).

$M^+$ 170/2; nmr ($CDCl_3$) 9.98 (s,1H).

Example F

1-Hydroxy-2-(trifluoromethyl)cyclohexanecarbonitrile

Trimethylsilylcyanide (3.6ml, 0.027moles) was added with stirring at 18° to a solution of 2-(trifluoromethyl)cyclohexanone (3g, 0.018moles) and zinc iodide (0.1g) in dichloromethane (40ml). After stirring overnight, the solution was poured onto cold dilute hydrochloric acid and the product extracted with dichloromethane (x3). The combined organic extracts were washed with brine, dried ($MgSO_4$) and the solvent evaporated to give 2-(trifluoromethyl)-1-(trimethylsilyloxy)-cyclohexanecarbonitrile (4.8g) as an oil.

This oil was dissolved in ethanol (50ml) and 10% aqueous sulphuric acid (15ml) and stirred for 1.5 hours. The ethanol was removed under reduced pressure and the residue treated with brine and ethyl acetate. The aqueous layer was separated and extracted with ethyl acetate (x3). The combined organic extracts were washed with brine, dried ($MgSO_4$) and the solvent evaporated to give the title compound (3.2g) as a mixture of epimers.

nmr ($CDCl_3$)$\delta$3.3 and 2.85 (2xbrs, 1H) and

2.5(m,1H).

Example G

2-(Trifluoromethyl)-1-cyclohexenecarbonitrile and 6-(trifluoromethyl)-1-cyclohexenecarbonitrile

1-Hydroxy-2-(trifluoromethyl)cyclohexane carbonitrile (16.3g, 0.084moles), phosphorous oxychloride (152ml, 1.7moles), pyridine (134ml, 1.7moles) and benzene (600ml) were heated at reflux for 6 hours. After cooling to room temperature the reaction mixture was cautiously added to ice. After 45 minutes the product was extracted with ether/ethyl acetate (x4). The combined organic extracts were washed with brine (x3), dried ($MgSO_4$) and the solvent evaporated. The two isomeric nitriles were separated by chromatography on silica eluting with ethyl acetate/petroleum ether (60-80$^o$) mixtures.

The 2-(trifluoromethyl)nitrile (3.3g) was eluted first ($M^+$175; nmr ($CDCl_3$) 2.45 and 2.35 (2xm, 2x2H) and 1.72 (m, 4H) followed by the 6-(trifluoromethyl)-nitrile (5.2g) ($M^+$175); nmr ($CDCl_3$) 7.0 (m,1H).

Example H

2-(Trifluoromethyl)-1-cyclohexenecarboxaldehyde

Diisobutylaluminium hydride (3.75ml of 1.4M in toluene, 5.3mmoles) was added to a stirred solution of

0174071

2-(trifluoromethyl)-1-cyclohexenecarboxaldehyde (0.45g, 2.6mmoles) in benzene (25ml) under nitrogen at 15°. After 2.5 hours, the solution was cooled in ice and 10% aqueous sulphuric acid was added. The product was extracted with ethyl acetate (x3) and the combined extracts were washed with brine, dried ($MgSO_4$) and the solvent evaporated to leave the desired aldehyde (0.25g). $M^+$178; nmr ($CDCl_3$) 10.1 (q,1H).

What we claim is:-

1. A compound of formula I,

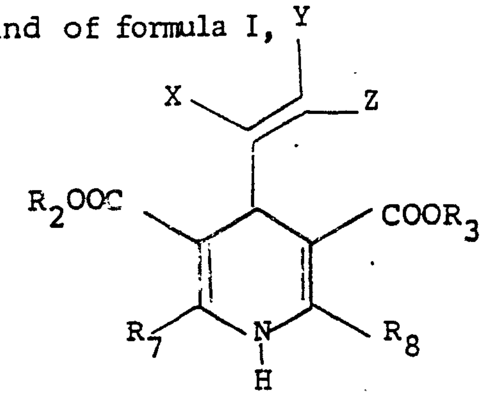

I

in which $R_2$ and $R_3$, which may be the same or different, each represent alkyl C1 to 6 or cycloalkyl C3 to 8,

$R_7$ and $R_8$, which may be the same or different, each represent alkyl C1 to 6 optionally substituted by halogen, and

X, Y and Z, which may be the same or different, are each hydrogen, halogen, nitro or alkyl C1 to 6 optionally substituted by halogen, or Z is as defined above and X and Y, together with the carbon atoms to which they are attached, form a C5 to 8 carbocyclic ring, other than a benzene ring,

provided that when $R_2$, $R_3$, $R_7$ and $R_8$ are all methyl and X and Z are hydrogen; or when $R_2$ and $R_3$ are both ethyl, $R_7$ and $R_8$ are both methyl and X and Z are hydrogen; then Y is other than methyl.

2. A compound according to claim 1, wherein $R_2$ and $R_3$ are selected from methyl and 1-methylethyl.

3. A compound according to claim 1 or 2, wherein $R_7$ and $R_8$ are selected from methyl and monofluoromethyl.

4. A compound according to any one of the preceding claims, wherein Z is chloro, bromo, methyl or $-CF_3$, Y is hydrogen or chloro, and X is hydrogen or chloro, or Y and Z together form a $-(CH_2)_3-$ or $-(CH_2)_4-$ chain.

5. A compound according to any one of claims 1 to 4, wherein X and Y, together with the carbon atoms to which they are attached, form a ring of formula,

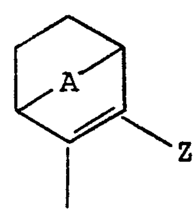

in which Z is as defined in claim 1, and
A is $-CH_2-$ or $-CH_2CH_2-$.

6. 3-Methyl 5-(1-methylethyl) 4-(2-chloro-1-cyclohexenyl)-2-(fluoromethyl)-1,4-dihydro-6-methyl-3,5-pyridinedicarboxylate,

3-Methyl 5-(1-methylethyl) 4-(2-chloro-1-cyclopentenyl)-2-(fluoromethyl)-1,4-dihydro-6-methyl-3,5-pyridinedicarboxylate,

3-Methyl 5-(1-methylethyl) 4-(2-chloro-1-cyclohexenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(2-methyl-1-cyclohexenyl)-3,5-pyridinedicarboxylate,

3-Methyl 5-(1-methylethyl) 4-(2,2-dichloroethenyl)-2-(fluoromethyl)-1,4-dihydro-6-methyl-3,5-pyridinedicarboxylate,

bis(1-methylethyl) 4-(2,2-dichloroethenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(1,2,2-trichloroethenyl)-3,5-pyridinedicarboxylate,

3-Methyl 5-(1-methylethyl) 4-(bicyclo[2.2.2]oct-2-en-2-yl)-2-(fluoromethyl)-1,4-dihydro-6-methyl-3,5-pyridine dicarboxylate,

3-Methyl 5-(1-methylethyl) 4-(3-chlorobicyclo [2.2.2]-oct-2-en-2-yl)-2-(fluoromethyl)-1,4-dihydro-6-methyl-3,5-pyridinedicarboxylate,

3-Methyl 5-(1-methylethyl) 4-(2-bromo-1-cyclohexenyl)-2-(fluoromethyl)-1,4-dihydro-6-methyl-3,5-pyridinedicarboxylate, or

3-Methyl 5-(1-methylethyl) 2-(fluoromethyl)-1,4-dihydro-6-methyl-4-(2-(trifluoromethyl)-1-cyclohexenyl-3,5-pyridinedicarboxylate.

7. A pharmaceutical formulation comprising a compound according to any one of the preceding claims in admixture

with a pharmaceutically acceptable adjuvant, diluent or carrier.

8. The use of a compound according to any one of claims 1 to 6 as a pharmaceutical.

9. The use of a compound according to any one of claims 1 to 6 to make a pharmaceutical formulation for use in the treatment of a cardiovascular condition.

10. A process for the production of a compound of formula I, as defined in claim 1, which comprises

a) reaction of a compound of formula II,

YZC=CXCHO II

either with a compound of formula III or with compounds of formulae III and IV,

$R_2OOCCH=C(R_7)NH_2$ III

$R_3OOCCH_2COR_8$ IV

in which formulae Y,Z,X, $R_2$, $R_3$, $R_7$ and $R_8$ are as defined in claim 1,

b) reaction of a compound of formula V,

$YZC=CX-CH=C(COOR_3)COR_8$ V

in which Y, Z, X, $R_3$ and $R_8$ are as defined in claim 1, with a compound of formula III, or

c) production of an optical isomer of a compound of formula I by resolution of a mixture of optical isomers of the compound.

1383J(ir)/jaa

What we claim is:-

1. A process for the production of a compound of formula I,

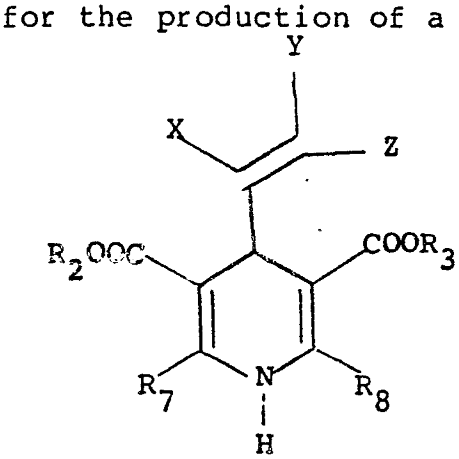

I

in which $R_2$ and $R_3$, which may be the same or different, each represent alkyl C1 to 6 or cycloalkyl C3 to 8,

$R_7$ and $R_8$, which may be the same or different, each represent alkyl C1 to 6 optionally substituted by halogen, and

X, Y and Z, which may be the same or different, are each hydrogen, halogen, nitro or alkyl C1 to 6 optionally substituted by halogen, or Z is as defined above and X and Y, together with the carbon atoms to which they are attached, form a C5 to 8 carbocyclic ring, other than a benzene ring,

provided that when $R_2$, $R_3$, $R_7$ and $R_8$ are all methyl and X and Z are hydrogen; or when $R_2$ and $R_3$ are both ethyl, $R_7$ and $R_8$ are both methyl and X and Z are hydrogen; then Y is other than methyl,

which comprises

a) reaction of a compound of formula II,

$YZC{=}CXCHO$ II

either with a compound of formula III or with compounds of formulae III and IV,

$R_2OOCCH{=}C(R_7)NH_2$ III

$R_3OOCCH_2COR_8$ IV

in which formulae Y,Z,X, $R_2$, $R_3$, $R_7$ and $R_8$ are as defined in claim 1,

b) reaction of a compound of formula V,

$YZC{=}CX{-}CH{=}C(COOR_3)COR_8$ V

in which Y, Z, X, $R_3$ and $R_8$ are as defined in claim 1, with a compound of formula III, or

c) production of an optical isomer of a compound of formula I by resolution of a mixture of optical isomers of the compound.

2. A process according to claim 1, wherein $R_2$ and $R_3$ are selected from methyl and 1-methylethyl.

3. A process according to claim 1 or 2, wherein $R_7$ and $R_8$ are selected from methyl and monofluoromethyl.

4. A process according to any one of the preceding claims, wherein Z is chloro, bromo, methyl or $-CF_3$, Y is hydrogen or chloro, and X is hydrogen or chloro, or Y and Z together form a $-(CH_2)_3-$ or $-(CH_2)_4-$ chain.

5. A process according to any one of claims 1 to 4, wherein X and Y, together with the carbon atoms to which

they are attached, form a ring of formula,

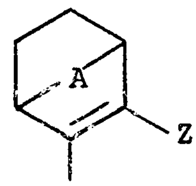

in which Z is as defined in claim 1, and

A is $-CH_2-$ or $-CH_2CH_2-$.

6. A process according to any one of the preceding claims, whereing the compound of formula I is

3-Methyl 5-(1-methylethyl) 4-(2-chloro-1-cyclohexenyl)-2-(fluoromethyl)-1,4-dihydro-6-methyl-3,5-pyridinedicarboxylate.

7. A process according to any one of claims 1 to 5, wherein the compound of formula I is

3-Methyl 5-(1-methylethyl) 4-(2-chloro-1-cyclopentenyl)-2-(fluoromethyl)-1,4-dihydro-6-methyl-3,5-pyridinedicarboxylate,

3-Methyl 5-(1-methylethyl) 4-(2-chloro-1-cyclohexenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(2-methyl-1-cyclohexenyl)-3,5-pyridinedicarboxylate,

3-Methyl 5-(1-methylethyl) 4-(2,2-dichloroethenyl)-2-(fluoromethyl)-1,4-dihydro-6-methyl-3,5-pyridinedicarboxylate,

bis(1-methylethyl) 4-(2,2-dichloroethenyl)-1,4-dihydro-2,6-dimethyl-3,5-pyridinedicarboxylate,

3-Methyl 5-(1-methylethyl) 2-(fluoromethyl)-1,4-dihydro-6-methyl-4-(1,2,2-trichloroethenyl)-3,5-pyridinedicarboxylate,

3-Methyl 5-(1-methylethyl) 4-(bicyclo[2.2.2]oct-2-en-2-yl)-2-(fluoromethyl)-1,4-dihydro-6-methyl-3,5-pyridine dicarboxylate,

3-Methyl 5-(1-methylethyl) 4-(3-chlorobicyclo [2.2.2]-oct-2-en-2-yl)-2-(fluoromethyl)-1,4-dihydro-6-methyl-3,5-pyridinedicarboxylate,

3-Methyl 5-(1-methylethyl) 4-(2-bromo-1-cyclohexenyl)-2-(fluoromethyl)-1,4-dihydro-6-methyl-3,5-pyridinedicarboxylate, or

3-Methyl 5-(1-methylethyl) 2-(fluoromethyl)-1,4-dihydro-6-methyl-4-(2-(trifluoromethyl)-1-cyclohexenyl-3,5-pyridinedicarboxylate.

8. The use of a compound according to any one of claims 1 to 7 to make a pharmaceutical formulation for use in the treatment of a cardiovascular condition.